# EUROPEAN PATENT SPECIFICATION

(11) **EP 2 970 359 B1**
(45) Date of publication and mention of the grant of the patent: **07.04.2021**
(21) Application number: 13878435.0
(22) Date of filing: 15.03.2013
(51) Int. Cl.: C07H 19/12, A61K 31/7068, A61K 31/706, A61P 35/00, A61P 35/02, A61P 43/00

(54) **FLUORINATED PYRIMIDINE ANALOGS AND METHODS OF USE THEREOF**
FLUORIERTE PYRIMIDINANALOGA UND VERFAHREN ZUR VERWENDUNG DAVON
ANALOGUES DE PYRIMIDINE FLUORÉS ET MÉTHODES D'UTILISATION CORRESPONDANTES

(43) Date of publication of application: 20.01.2016
(73) Proprietor: Epigenetics Pharma LLC, Mercer Island WA 98040 (US)
(72) Inventor: Daifuku, Richard, Mercer Island, WA 98040 (US); Gall, Anna Stanislav, Woodinville, WA 98072 (US); Sergueev, Dmitri S., Kirkland, WA 98034 (US)
(74) Representative: Gevers Patents
(86) International application number: PCT/US2013/032593
(87) International publication number: WO 2014/143051

(56) References cited:
- WO-A1-2012/106299
- WO-A2-2006/063111
- WO-A2-2008/028193
- US-A1- 2006 128 653
- US-A1- 2011 042 247
- US-A1- 2013 001 818
- US-B1- 6 933 287

## Description

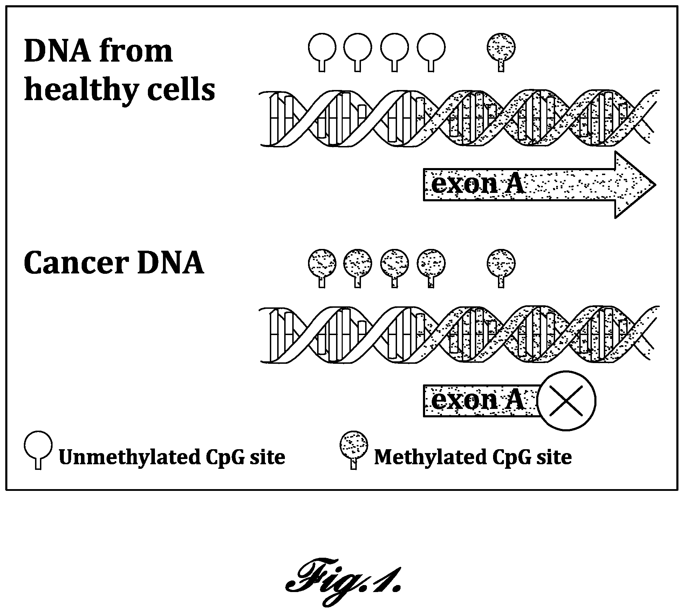

Aberrant DNA methylation is an epigenetic mechanism that can inactivate the expression of genes that suppress tumorigenesis. The genes involved include tumor suppressor genes; genes that suppress apoptosis, metastasis and angiogenesis; genes that repair DNA; and genes that express tumor-associated antigens. The molecular mechanism of silencing gene expression appears to be due to the attachment of 5-methylcytosine binding proteins to the methylated promoter, which blocks the action of transcription factors (see FIGURE 1).

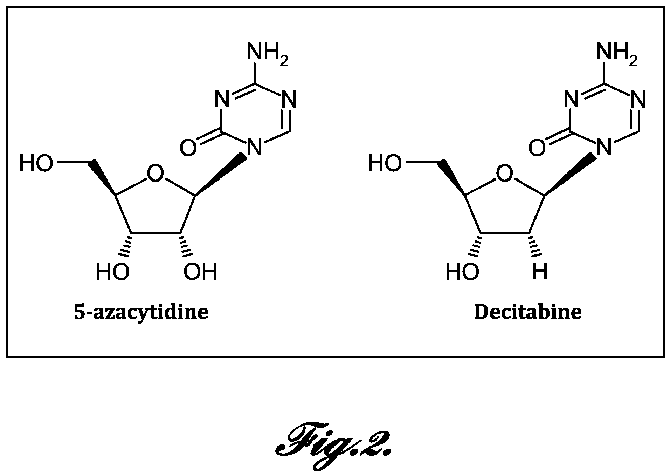

Because this epigenetic change is reversible, it presents an interesting target for chemotherapeutic intervention. 5-Azacytidine was the first hypomethylating agent approved by the FDA for the treatment of a neoplasm and the deoxy-analog, 5-azadeoxycytidine or decitabine, was more recently approved for the same indication (see FIGURE 2). Both drugs produce remissions or clinical improvements in more than half of the treated patients with myelodysplatic syndrome (MDS). Features of responses include the requirement for multiple cycles of therapy, slow responses, and actual clonal elimination. Optimization of therapy has included (1) reducing the dose to favor hypomethylation over cytotoxicity, (2) prolonging administration schedules, and (3) increasing dose intensity without reaching cytotoxicity. Molecularly, hypomethylation and gene reactivation have been shown and seem to be required for responses. The data in MDS represent a proof-of-principle for epigenetic therapy. Although the therapy is effective, with complete responses lasting months to years in some patients, resistance seems to develop in the majority of patients, and the mechanisms of resistance are unknown.

Data from the two currently approved drugs suggest that myeloid malignancies are the neoplasms most sensitive to inhibitors of DNA methylation. However, there is no known reason why solid tumors should not respond as well. Recently, preclinical studies with DNA methyl transferase inhibitors (DNMTIs) have demonstrated that they are also potent angiostatic agents, inhibiting tumor endothelial cells and angiogenesis in vitro and in vivo, adding to the rationale for the treatment of solid tumors with these agents.

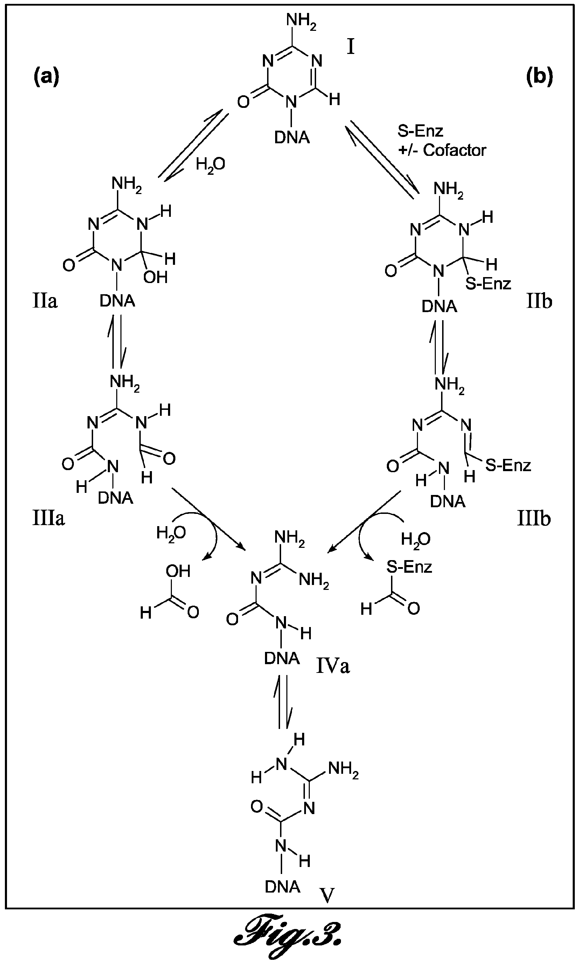

5-Azacytidine and decitabine are known to be unstable in water, with cleavage of the base at the 6-position of the cytosine ring (see FIGURE 3, IIIa). Both nucleosides are known to suffer from a short half-life. The half-life of 5-azacytidine following subcutaneous administration is reported to be 41 minutes and that of decitabine 30 minutes. Both drugs are good substrates for cytidine deaminase and the short half-life is primarily due to deamination of the base to inactive uracil.

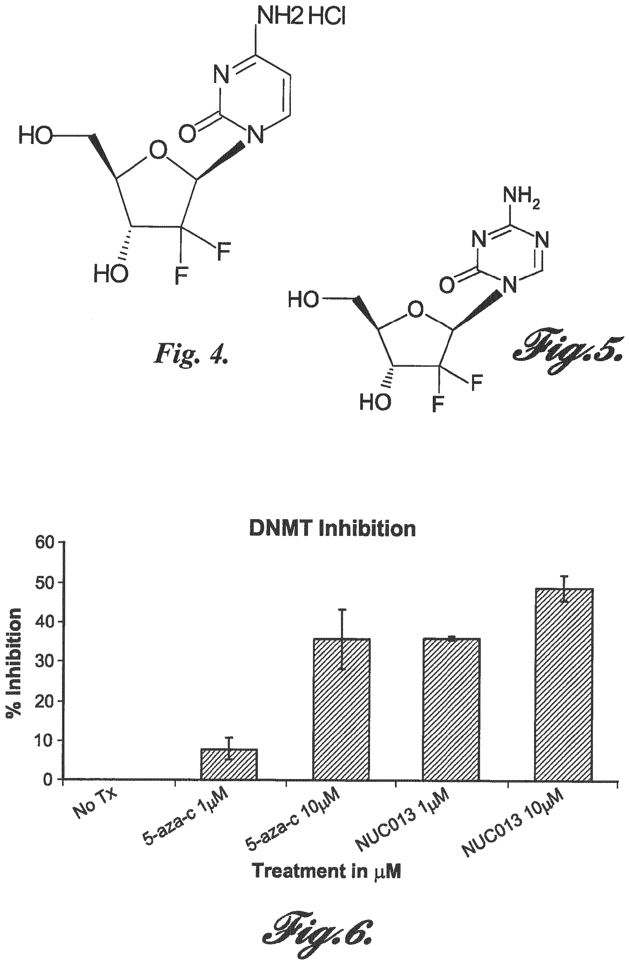

Gemcitabine differs from cytidine by a difluorinated sugar at the 2'-position. See FIGURE 4.

The cytotoxic effect of gemcitabine is attributed to a combination of two actions of the diphosphate and the triphosphate nucleosides, which leads to inhibition of DNA synthesis. First, gemcitabine diphosphate inhibits ribonucleotide reductase, which is responsible for catalyzing the reactions that generate the deoxynucleoside triphosphates for DNA synthesis. Inhibition of this enzyme by the diphosphate nucleoside causes a reduction in the concentrations of deoxynucleotides, including dCTP. Second, gemcitabine triphosphate competes with dCTP for incorporation into DNA. The reduction in the intracellular concentration of dCTP (by the action of the diphosphate) enhances the incorporation of gemcitabine triphosphate into DNA (self-potentiation). After the gemcitabine nucleotide is incorporated into DNA, only one additional nucleotide is added to the growing DNA strands. After this addition, there is inhibition of further DNA synthesis. DNA polymerase epsilon is unable to remove the gemcitabine nucleotide and repair the growing DNA strands (masked chain termination).

There exists a need for novel cancer therapeutics with improved efficacy, safety, and/or pharmacokinetic profiles. For instance, WO 2006/063111 discloses pharmaceutical formulations of cytidine analogs and derivatives, such as 5-azacytidine but does not show any therapeutic uses of the formulations in a treatment of a disease or disorder. WO 2008/028193 discloses an oral formulation of a cytidine analog, including, 5-azacytidine but does not provide any data showing diseases or disorders that may be treated by the exemplified compositions. WO 2012/106299 discloses pharmaceutical compositions comprising cytidine analogs for parenteral administration but does not provide any data showing therapeutic uses of the compositions in a treatment of a disease or disorder.

The invention provides novel fluorinated pyrimidine analogs and compositions for the treatment of cancer.

### SUMMARY OF THE INVENTION

In one aspect, the invention provides fluorinated pyrimidine analogs.

In one embodiment, the invention provides 2',2'-difluoro-5-azadeoxycytidine, its anomers, and pharmaceutically acceptable salts thereof for use in the treatment of a solid tumor carcinoma or of a hematologic malignancy as recited in claim 1 or 3.

In another embodiment, the invention provides 2',2'-difluoro-5,6-dihydro-5-azadeoxycytidine, its anomers, and pharmaceutically acceptable salts thereof as recited in claim 6.

In a further embodiment, the invention provides 2'-deoxy-2',2'-difluorozebularine, its anomers, and pharmaceutically acceptable salts thereof as recited in claim 10 and their use in the treatment of a solid tumor carcinoma or of a hematologic malignancy as recited in claim 12.

In another aspect, the invention provides pharmaceutical composition having the features of claim 7 or 11.

The subject can be a human subject.

### BRIEF DESCRIPTION OF THE DRAWINGS

FIGURE 1 is a schematic illustration of overexpression of DNA methyl transferase (DNMT), as occurs in cancer cells, can lead to hypermethylation and inactivation of promoters of tumor suppression and growth regulatory genes.
FIGURE 2 illustrates the chemical structures of 5-azacytidine and decitabine.
FIGURE 3 illustrates reaction pathways for ring opening and hydrolysis of 5-azacytosine residue in DNA in solution (a) and after covalent linkage to the active site of a DNMT (b).
FIGURE 4 illustrates the chemical structure of gemcitabine HCl.
FIGURE 5 illustrates the chemical structure of 2',2'-difluoro-5-azadeoxycytidine (NUC013).
FIGURE 6 compares DNMT inhibition in colon cancer cells (HCT116) of NUC013 and 5-azacytidine.
FIGURE 7 compares the activity of NUC013 and decitabine (DAC) on the THP-1 leukemia cell line.
FIGURE 8 compares the activity of NUC013 and decitabine (DAC) on the Kasumi-1 leukemia cell line.
FIGURE 9 illustrates Western blot comparing inhibitory activity against DNMT1 of decitabine and NUC013 in THP-1 and Kasumi-1 cell lines.
FIGURE 10 compares Giemsa stain of THP-1 cells treated with decitabine or NUC013 compared to vehicle control.
FIGURE 11 compares Giemsa stain of Kasumi-1 cells treated with decitabine or NUC013 compared with vehicle control.
FIGURE 12 illustrates the chemical structure of 2',2'-difluoro-5,6-dihydro-5-azadeoxycytidine (NUC014).
FIGURE 13 illustrates the chemical structure of 2'-deoxy-2',2'-difluorozebularine (NUC019).

### DETAILED DESCRIPTION OF THE INVENTION

The present invention provides therapeutic drug compounds that are useful in the treatment of cancer. The therapeutic drug compounds of the invention are fluorinated pyrimidine analogs. The present invention also provides compositions that include the fluorinated pyrimidine analogs, methods for making the fluorinated pyrimidine analogs , and methods for treating cancer using the fluorinated pyrimidine analogs .

The therapeutic drug compounds are a family of DNA methyl transferase inhibitors (DNMTIs) that substitute the sugar moiety from gemcitabine for the natural sugar.

In one aspect, the present invention provides fluorinated pyrimidine analogs. Representative fluorinated pyrimidine analogs of the invention include:
2',2'-difluoro-5-azadeoxycytidine (referred to herein as "NUC013");
2',2'-difluoro-5,6-dihydro-5-azadeoxycytidine (referred to herein as "NUC014"); and
2'-deoxy-2',2'-difluorozebularine (referred to herein as "NUC019").

In one embodiment, the invention provides 2',2'-difluoro-5-azadeoxycytidine, its anomers (α and β), and salts thereof.

In another embodiment, the invention provides 2',2'-difluoro-5,6-dihydro-5-azadeoxycytidine, its anomers (α and β), and salts thereof.

In a further embodiment, the invention provides 2'-deoxy-2',2'-difluorozebularine, its anomers (α and β), and salts thereof.

Suitable salts include pharmaceutically acceptable salts. Representative salts include acetate, adipate, aspartate, benzoate, benzene sulfonate (besylate), bisulfate, butyrate, citrate, camphorate, camphorsulfonate, cyclopentane propionate, digluconate, dodecylsulfate, 1,2-ethane disulfonate (edisylate), ethane sulfonate (esylate), formate, fumarate, glucoheptanoate, glycerophosphate, glycolate, hemisulfate, heptanoate, hexanoate, hydrochloride, hydrobromide, hydroiodide, 2-hydroxyethane sulfonate, lactate, maleate, methane sulfonate (mesylate), 2-naphthalene sulfonate (napsylate), nicotinate, nitrate, oxalate, palmoate, pectinate, persulfate, 3-phenylpropionate, phosphate, picrate, pivalate, propionate, salicylate, succinate, sulfate, tartrate, thiocyanate, tosylate, and undecanoate salts.

In another aspect, the present invention provides pharmaceutical compositions comprising at least one fluorinated pyrimidine analog of the invention together with a pharmaceutically acceptable carrier or diluent suitable for administration to a human or animal subject, either alone or together with other therapeutics and/or anticancer agents. The fluorinated pyrimidine analogs of the invention may be formulated into a composition that additionally comprises suitable pharmaceutically acceptable carriers, including excipients and other compounds that facilitate administration of the fluorinated pyrimidine analogs to a mammalian subject. The pharmaceutical compositions are useful for the administration of fluorinated pyrimidine analogs to treat cancer.

Compositions that include one or more fluorinated pyrimidine analogs of the invention are administered to deliver therapeutically effective amounts of the fluorinated pyrimidine analog. Therapeutically effective amounts of the fluorinated pyrimidine analog(s) will generally range up to the maximally tolerated dosage, but the concentrations are not critical and may vary widely. The precise amounts employed by the attending physician will vary, of course, depending on the compound, route of administration, physical condition of the patient and other factors. The daily dosage may be administered as a single dosage or may be divided into multiple doses for administration. Administration of the fluorinated pyrimidine analogs of the invention is accomplished by any effective route, for example, parenterally or orally.

The amount of the fluorinated pyrimidine analogs of the invention actually administered will be a therapeutically effective amount, which term is used herein to denote the amount needed to produce a substantial beneficial effect. Effective doses may be extrapolated from dose-response curves derived from in vitro or animal model test systems. The animal model is also typically used to determine a desirable dosage range and route of administration. Such information can then be used to determine useful doses and routes for administration in humans or other mammals. The determination of an effective dose is well within the capability of those skilled in the art. Thus, the amount actually administered will be dependent upon the individual to which treatment is to be applied, and will preferably be an optimized amount such that the desired effect is achieved without significant side-effects.

In another aspect of the invention, methods for making the fluorinated pyrimidine analogs are provided. The preparation of 2',2'-difluoro-5-azadeoxycytidine (NUC013) is provided in EXAMPLE 1. The preparation of 2',2'-difluoro-5,6-dihydro-5-azadeoxycytidine (NUC014) is provided in EXAMPLE 2. The preparation of 2',2'-difluorozebularine (NUC019) is provided in EXAMPLE 3.

In another aspect, the present invention provides methods of treating human or animal subjects suffering from a cellular proliferative disease, such as cancer, using the fluorinated compounds of the invention. Representative cell proliferative diseases treatable by the compounds of the invention include hematologic cancers, such as leukemia, lymphoma, and myeloma, and nonhematologic cancers, such as solid tumor carcinomas (e.g., breast, ovarian, pancreatic, colon, colorectal, non-small lung, renal, and bladder), sarcomas, and gliomas. The present invention provides methods of treating a human or animal subject in need of such treatment, comprising administering to the subject a therapeutically effective amount of one or more fluorinated pyrimidine analogs of the invention, either alone or in combination with one or more other therapeutic and/or anticancer agents.

### Representative fluorinated pyrimidine compounds

The primary rationale for the development of this family of molecules is based on the mechanism of self-potentiation noted with gemcitabine, though such molecules may present other interesting characteristics.

NUC013. 2',2'-difluoro-5-azadeoxycytidine (NUC013) (see FIGURE 5) is a novel cytidine analog, related to 5-azacytidine and decitabine, hence producing known metabolites. Having known metabolites facilitates clinical development by decreasing the risk of unforeseen toxicities. For example, metabolite toxicity has complicated the clinical development of 5-fluorodeoxycytidine as a DNMTI.

NUC013 has been screened for activity in cell lines and compared to 5-azacytidine and decitabine. See Table 1.

**Table 1. In vitro cytotoxicity of NUC013 compared to decitabine and 5-azacytidine.**

| Compound | Breast MDA-MB-231 | NSCL NCI-H460 | Colon HCT-116 | Leukemia L1210 | Renal ACHN* | Ovarian OVCAR-8* | Leukemia P388* |
|---|---|---|---|---|---|---|---|
| | (µM) | | (µM) | (µM) | (µM) | | (µM) |
| | | (µM) | | | | (µM) | |
| Decitabine | >62.5 | 13-44 | >62.5 | 0.24 | >1000 | >1000 | 0.0004 |
| Azacytidine | 1.64 | 0.43 | 0.62 | 1.7-2.4 | 2.51 | 6.38 | 0.74 |
| NUC013 | >62.5 | 4.39 | 4.54 | 1.02 | 0.89 | 25.88 | 0.48 |

As can be seen from Table 1, the cytotoxicity of NUC013 against various tumor cells lines, both solid and hematologic, has a unique profile. NUC013 is active against cell lines that are resistant to decitabine, particularly solid tumor cell lines.

NUC013 has also been tested for DNA methyl transferase inhibition (see FIGURE 6). In colon cancer cells, NUC013 is approximately 10-fold more potent than 5-azacytine as a DNMTI. However, as 5-azacytidine is more cytotoxic than NUC013 in this cell line (GI₅₀ of 0.62 µM versus 4.54 µM, respectively), it is possible to achieve substantial inhibition of DNMT at drug concentrations that are less cytotoxic with NUC013 compared to 5-azacytidine.

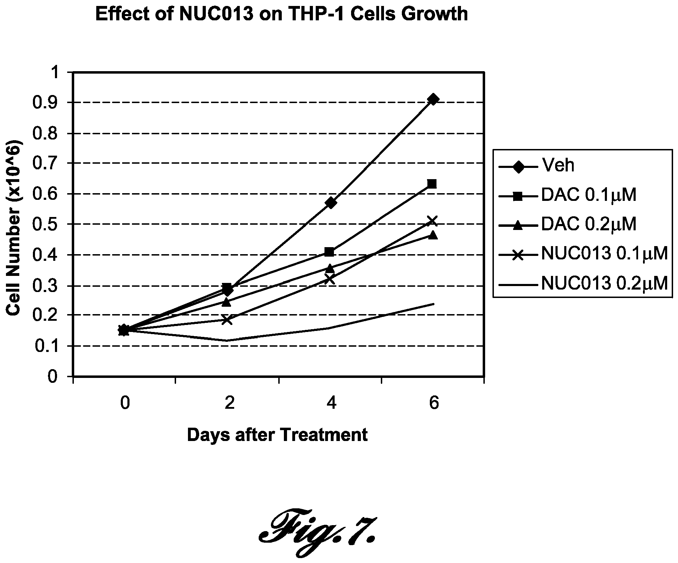

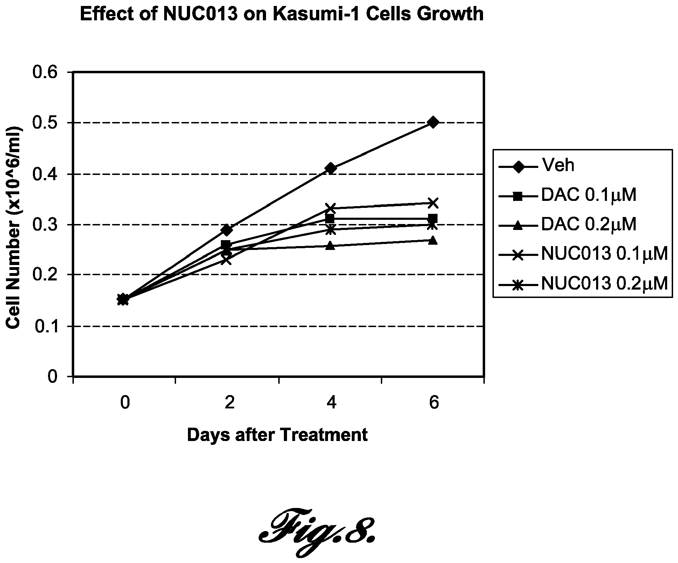

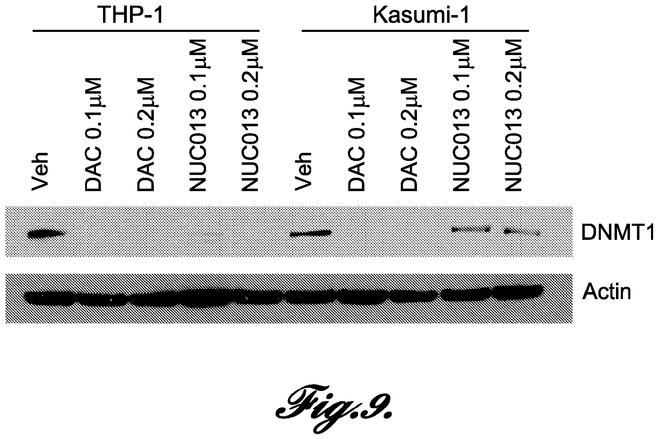

FIGURE 7 compares the activity of NUC013 with that of decitabine on leukemia cell lines THP-1 and Kasumi 1. FIGURE 8 compares the activity of NUC013 and decitabine (DAC) on the Kasumi-1 leukemia cell line. NUC013 displayed a stronger inhibitory effect on THP-1 leukemia cells growth than decitabine, but a slightly weaker effect in Kasumi-1 cells at equimolar concentrations. FIGURE 9 illustrates Western blot comparing inhibitory activity against DNMT1 of decitabine and NUC013 in THP-1 and Kasumi-1 cell lines.

In comparisons with decitabine, depletion of DNMT1 by NUC013 appears to be related to the cytoreduction demonstrated in FIGURES 5 and 6. Indeed, at the two concentrations tested in FIGURE 7, NUC013 demonstrates similar DNMT inhibition by Western blot compared with equimolar decitabine in THP-1 cells, while demonstrating stronger cytoreduction, but has weaker DNMT1 inhibition on Kasumi-1 cells correlating with weaker cytoreduction. These data further confirm that NUC013 is a DNMT inhibitor.

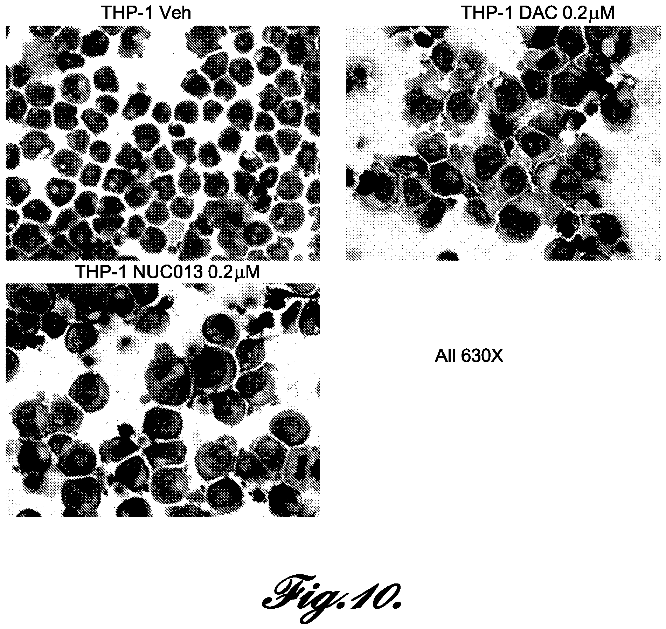

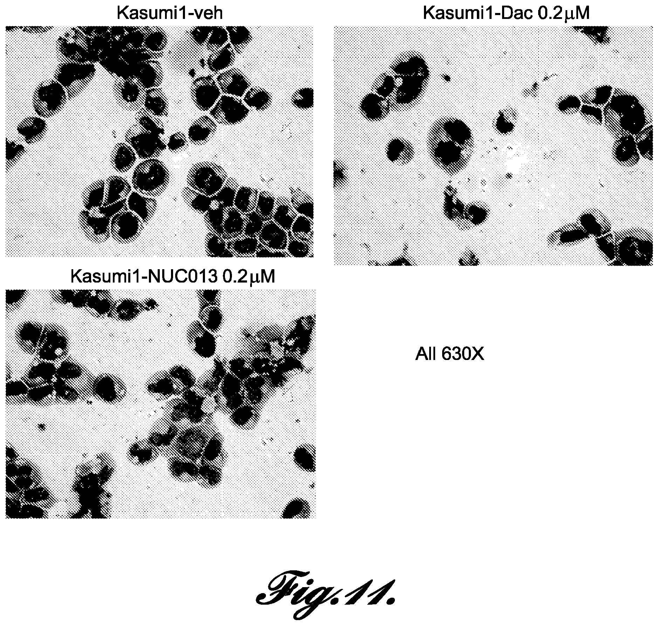

FIGURE 10 compares Giemsa stain of THP-1 cells treated with decitabine or NUC013 compared to vehicle control. FIGURE 11 compares Giemsa stain of Kasumi-1 cells treated with decitabine or NUC013 compared with vehicle control.

As demonstrated in FIGURES 8 and 9, treatment of THP-1 or Kasumi-1 cells by NUC013 or decitabine results in morphologic changes suggestive of cell differentiation, with greater effects observed for NUC013 treatment of THP-1 than Kasumi-1 cells, compatible with greater DNMT1 inhibition in THP-1 cells.

The preparation of 2',2'-difluoro-5-azadeoxycytidine (NUC013) is described in Example 1. In vitro cytotoxicity of NUC013 (non-small cell lung, colorectal, leukemia, and breast cancer) is described in Example 4. In vitro DNA methyltransferase inhibition of NUC013 is described in Example 5.

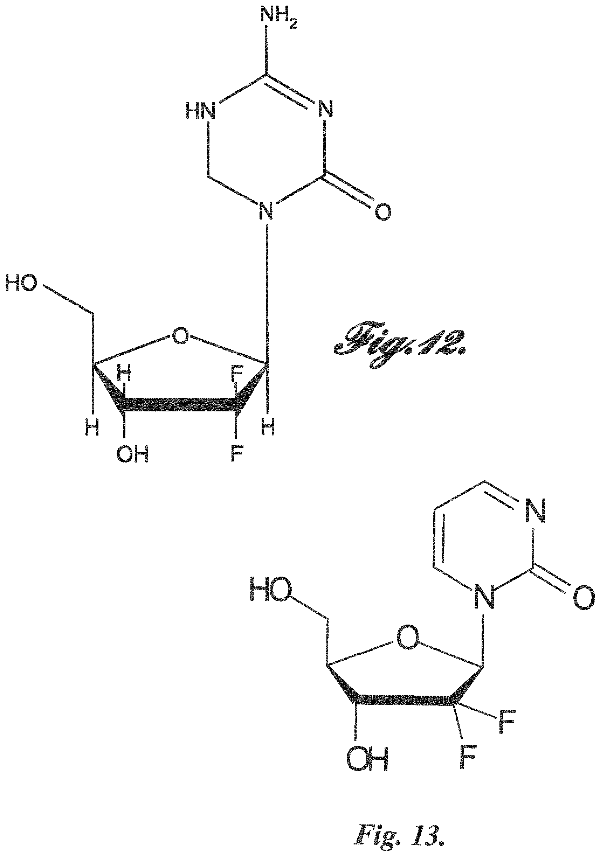

NUC014. NUC014 is the 5,6-dihydro derivative of NUC013. FIGURE 12 illustrates the chemical structure of 2',2'-difluoro-5,6-dihydro-5-azadeoxycytidine (NUC014). It has been found to be stable in an aqueous environment and does not undergo hydrolytic cleavage at the 6-position of the cytosine. Representative in vitro cytotoxicity of NUC014 is summarized in Table 2.

**Table 2. In vitro cytotoxicity of NUC014.**

| Compound | Breast MDA-MB-231 | NSCL NCI-H460 | Colon HCT-116 | Leukemia L1210 |
|---|---|---|---|---|
| | (µM) | (µM) | (µM) | (µM) |
| NUC014 | >31.25 | >31.25 | >31.25 | 90.58 |

NUC014 demonstrates moderate cytotoxicity against leukemia cell line L1210. Based on available data showing lesser cytotoxicity for 5,6-dihydro-5-aza-2'-deoxycytidine (DHADC) compared to decitabine, it is not surprising that NUC014 is also less cytotoxic than NUC013.

The preparation of 2',2'-difluoro-5,6-dihydro-5-azadeoxycytidine (NUC014) is described in Example 2.

NUC019 and NUC020. NUC019 is a derivative of zebularine. FIGURE 13 illustrates the chemical structure of 2'-deoxy-2',2'-difluorozebularine (NUC019). Representative in vitro cytotoxicity of zebularine and zebularine derivatives NUC019 and its α-anomer NUC020 is summarized in Table 3.

**Table 3. In vitro cytotoxicity of zebularine and zebularine derivatives**

| Compound | Breast MDA-MB-231 (µM) | NSCL NCI-H460 | Colon HCT-116 | Leukemia L1210 |
|---|---|---|---|---|
| | | (µM) | (µM) | (µM) |
| Zebularine | 143.92 | 99.08 | 47.88 | 108.27 |
| NUC019 | 165.90 | 135.65 | 310.26 | 320.95 |
| NUC020 | 212.92 | 135.94 | 278.69 | 346.21 |

The preparation of 2'-deoxy-2',2'-difluorozebularine (NUC019) is described in Example 3. In vitro cytotoxicity of NUC019 (non-small cell lung, colorectal, leukemia, and breast cancer) is described in Example 4.

The following examples are provided for the purpose of illustrating, not limiting, the invention.

### EXAMPLE 1

### Preparation of 2',2'-Difluoro-5-azadeoxycytidine (NUC013)

Two alternative pathways, shown in Scheme I, were used to prepare 2',2'-difluoro-5-azadeoxycytidine (Compound IV) (NUC013).
A. Preparation of 3',5'-dibenzoyl-2',2'-difluoro-5-azadeoxycytidine (Compound III) via 1-bromo-2-deoxy-2,2-difluoro-D-ribofuranosyl-3,5-dibenzoate (Compound I).
   (1) The starting material 2-deoxy-2,2-difluoro-D-ribofuranosyl-3,5-dibenzoate known in the art (Chou, et. al., Synthesis 1992, v.p.565-570) was converted to 1-bromo analog by a method similar to the one described in International Patent Application WO 2006/070985. The starting ribose (3.9 g, 10.3 mmol) was dissolved in 31 ml of toluene and dry triethylamine (1.43 ml, 10.3 mmol) was added. The solution was cooled to 0 °C and diphenyl phosphoryl chloride (2.64 ml, 12.3 mmol) in 8 ml of toluene was added during 15 min. The reaction mixture was warmed up to room temperature and incubated for 3.5 hours. The reaction was quenched by addition of 1 M HCl (10.2 ml), the toluene layer was separated and aqueous layer was extracted back with 10 ml ether. The organic layers were combined, extracted consequently with water, saturated NaHCO₃, and saturated NaCl (each 20 ml) and dried over MgSO₄. The solvents were removed and the product was isolated by flash chromatography on silica gel in hexane-ethyl acetate gradient. Yield 4.6 g, 73%.
   (2) To the intermediate diphenylphosphate analog (4.6 g, 7.5 mmol) prepared as described in Step (1), was added HBr in acetic acid (30%, 16.2 ml, 81.3 mmol) and the reaction mixture was incubated for 6.5 h at room temperature. The reaction mixture was diluted with 80 ml of methylene chloride and extracted twice with ice water, saturated NaHCO₃, and saturated NaCl (each 100 ml). The organic layer was dried with MgSO₄, filtered and evaporated to yield 1-bromo-2-deoxy-2,2-difluoro-D-ribofuranosyl-3,5-dibenzoate (Compound I, 3.1 g, 93%), which is a 9:1 mixture of α and β isomers.
   (3) 5-Azacytosine (98%, Aldrich, 5.0 g, 44.8 mmol) and ammonium sulfate (25 mg) were suspended in hexamethyldisilazane (25 ml) and chlorotrimethylsilane (0.2 ml) was added. The reaction mixture was heated at the reflux for 17 h. The clear solution was cooled, evaporated, coevaporated twice with dry xylene and vacuum dried to yield whitish solids of the silylated 5-azacytosine (∼7 g) which is used in whole for the next glycosylation step.
   (4) For glycosylation step 1-bromo-2-deoxy-2,2-difluoro-D-ribofuranosyl-3,5-dibenzoate (Compound I) (0.78 g, 1.77 mmol), prepared as described in Step (2), was dissolved in 4 ml anisole and transferred to the silylated 5-azacytosine solids prepared as in Step (3). The suspension was heated to 150 °C and 2 ml of anisole was added to complete dissolution of all solids. After 4 hours the reaction mixture was cooled and SnCl₄ (0.2 ml, 2 mmol) was added. The reaction mixture was reheated to 150 °C for 6 hours, cooled to room temperature, quenched by addition of methylene chloride (30 ml), methanol (10 ml) and silica gel (20 g) and dried to yield 3',5'-dibenzoyl-2',2'-difluoro-5-azadeoxycytidine (Compound III). The resulting powder was applied on the top of the packed silica gel column and products were isolated by flash chromatography in chloroform-methanol gradient. β-Isomer was eluted first followed by α-isomer (only partial separation was achieved, yield for β-isomer 12%, α-isomer 5%). Complete isomer separation was achieved by RP HPLC on Gemini C18 5u (21.2x250 mm column) in 50 mM triethylammonium acetate (pH 7.5)-acetonitrile gradient.
      ¹H NMR in CDCl₃ for compound III β-isomer: 8.12 ppm (s, 1H, H6), 7.8-8.1 (m, 4H, benzoyl), 7.4-7.6 (m, 2H, benzoyl), 7.2-7.4 (m, 4H, benzoyl), 6.38 (br.t, J=8.0 Hz, 1H, H1'), 5.65 (m, 1H, H3'), 4.70 (m, 2H, H5'), 4.58 (m, 1H, H4'). ESI MS: 473.3 [M+H]+, 471.1 [M-H]⁻.
B. Preparation of 2',2'-difluoro-5-azadeoxycytidine (Compound IV). 3',5'-dibenzoyl-2',2'-difluoro-5-azadeoxycytidine (Compound III) (15 mg, 3:1 mixture of α and β isomers), prepared as in Step A, was dissolved in 2 ml of anhydrous MeOH and 1 M NaOMe in MeOH (0.1 ml) was added. After 1 h at room temperature, the reaction mixture was evaporated and the deprotected isomers were isolated by RP HPLC on Gemini C18 5u (21.2x250 mm column) in 50 mM triethylammonium acetate (pH 7.5)-acetonitrile gradient. Immediately after separation fractions corresponding to β isomer were pooled and evaporated at below 10 °C to yield 0.4 mg (19%) of 2',2'-difluoro-5-azadeoxycytidine (Compound IV).
   ¹H NMR in DMSO-d6 for compound IV β-isomer: 8.48 ppm (s, 1H, H6), 7.79 (br, 2H, NH₂), 6.35 (br, 1H, 3'-OH), 6.07 t, J=8.0 Hz, 1H, H1'), 5.30 (br, 1H, 5'-OH), 4.90 (br, 1H, 5'-OH), 4.23 (br. m, 1H, H3'), 3.85 (m, 1H, H4'), 3.78 (m, 1H, H5'), 3.63 (m, 1H, H5'), UV 240 nm (sh) in 50 mM triethylammonium acetate (pH 7.5).
C. Preparation of 3',5'-dibenzoyl-2',2'-difluoro-5-azadeoxycytidine (Compound III) via 1-methylsulfonyl-2-deoxy-2,2-difluoro-D-ribofuranosyl-3,5-dibenzoate (Compound II).
   Silylated 5-azacytosine (2.3 g, 9 mmol) prepared as described in Step A(3), was dissolved in 2 ml of anisole at 130 °C. 1-Methylsulfonyl-2-deoxy-2,2-difluoro-D-ribofuranosyl-3,5-dibenzoate (Compound II) (0.4 g, 0.87 mmol), prepared as described in the art (Chou, et. al., Synthesis v.p.565-570, 1992), was dissolved in 1 ml of anisole and was added to the hot solution of the silylated 5-azacytosine. The reaction mixture was incubated at 150 °C for 7 hours, cooled to room temperature, quenched by addition of 15 ml methylene chloride, 15 g silica gel and 5 ml methanol and the suspension was dried on vacuum. The resulting powder was applied on the top of the packed silica gel column and products were isolated by flash chromatography in chloroform-methanol gradient. Appropriate fractions were pooled and evaporated to yield 3',5'-dibenzoyl-2',2'-difluoro-5-azadeoxycytidine (Compound III) as 2:1 mixture of α- and β-isomers (100 mg, 25% yield).
D. Preparation of 2',2'-difluoro-5-azadeoxycytidine (Compound IV). 3',5'-dibenzoyl-2',2'-difluoro-5-azadeoxycytidine (Compound III) (80 mg, 2:1 mixture of α and β isomers), prepared as in Step C, was dissolved in 2 ml of anhydrous MeOH and 1 M NaOMe in MeOH (0.1 ml) was added. After 1 h at room temperature, the reaction mixture was evaporated and the deprotected isomers were isolated by RP HPLC on Gemini C18 5u (21.2x250 mm column) in 50 mM triethylammonium acetate (pH 7.5)-acetonitrile gradient. Immediately after separation fractions corresponding to β isomer were pooled, evaporated at below 10 °C to yield 2.1 mg (13%) of 2',2'-difluoro-5-azadeoxycytidine (Compound IV).

RP HPLC retention time and spectral characteristics (i.e. 1H NMR and UV) for Compound IV were the same as in Step B. ESI MS: 265.2 [M+H]⁺.

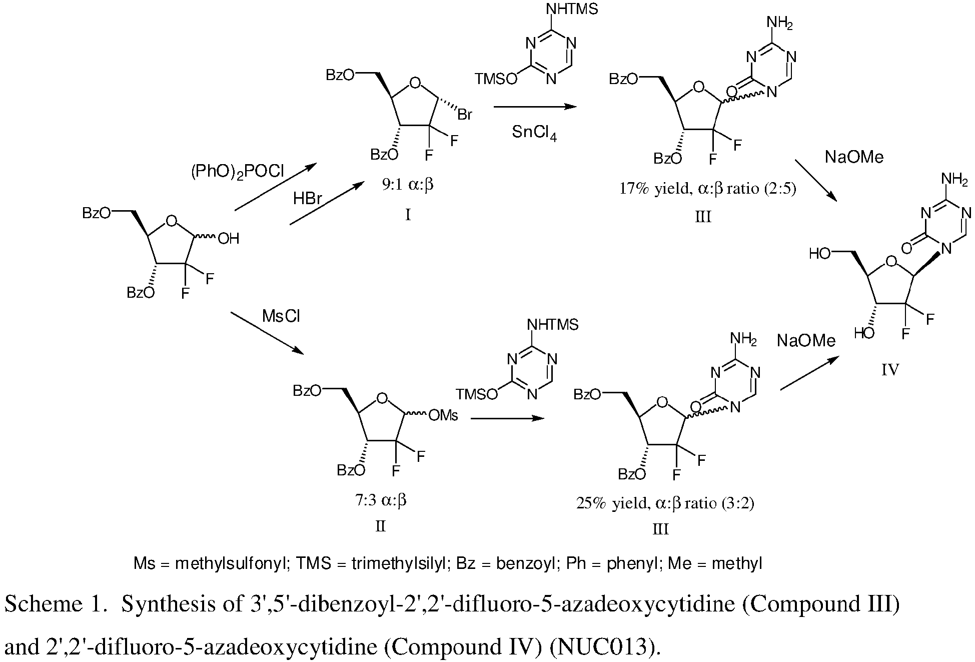

### EXAMPLE 2

### Preparation of 2',2'-Difluoro-5,6-dihydro-5-azadeoxycytidine (NUC014)

Preparation of 2',2'-difluoro-5,6-dihydro-5-azadeoxycytidine (Compound VI) (NUC014) is shown in Scheme 2.
A. Preparation of 3',5'-dibenzoyl-2',2'-difluoro-5-azadeoxycytidine (Compound V). Pure β-Isomer of 3',5'-dibenzoyl-2',2'-difluoro-5-azadeoxycytidine (Compound III) (144 mg, 0.30 mmol), prepared as in Example 1, was dissolved in 3 ml of methylene chloride and triethylamine (0.12 ml) and chlorotrimethysilane (0.1 ml) were added. After 0.5 hour the reaction mixture was diluted with 5 ml acetic acid and sodium borohydride (100 mg, 2.6 mmol) was added as powder. After 1 h at room temperature the reaction mixture was evaporated and separated by RP HPLC on Gemini C18 5u (21.2x250 mm column) in 50 mM triethylammonium acetate (pH 7.5)-acetonitrile gradient. Fractions corresponding to the product were pooled and evaporated to yield 83 mg (57%) of 3',5'-dibenzoyl-2',2'-difluoro-5-azadeoxycytidine (Compound V).
   ¹H NMR in DMSO-d6 for compound V β-isomer: 7.93-8.07 (m, 4H, benzoyl), 7.64-7.77 (m, 2H, benzoyl), 7.47-7.62 (m, 4H, benzoyl), 6.08 (t, J=7.2 Hz, 1H, H1'), 5.63 (m, 1H, H3'), 4.55-4.70 (m, 3H, H4' and H5'), 4.48 (s, 2H, CH₂). ESI MS: 475.4 [M+H]+.
B. Preparation of 2',2'-difluoro-5,6-dihydro-5-azadeoxycytidine (Compound VI). 3',5'-dibenzoyl-2',2'-difluoro-5-azadeoxycytidine (Compound V) (80 mg, 0.168 mmol), prepared as in Step A, was dissolved in 6 ml of anhydrous MeOH and 1 M NaOMe in MeOH (0.3 ml) was added. After 1 h at room temperature, the reaction mixture was quenched by addition of acetic acid (0.02 ml), evaporated and the deprotected product was isolated by RP HPLC on Gemini C18 5u (21.2x250 mm column) in 50 mM triethylammonium acetate (pH 7.5)-acetonitrile gradient. Appropriate fractions were pooled and evaporated to yield 49 mg (95% yield) of 2',2'-difluoro-5,6-dihydro-5-azadeoxycytidine (Compound VI).
   ¹H NMR in DMSO-d6 for compound VI: 5.87 (dd, J₁=9.6 Hz, J2=10.8 Hz, 1H, H1'), 4.51 and 4.29 (AB, 2H, CH₂), 3.98 (ddd, J₁=8.4 Hz, J2=12.8 Hz, J3=12.8 Hz, 1H, H3'), 3.67 (m, 1H, H5'), 3.58 (m, 1H, H4'), 3.52 (m, 1H, H5'). ESI MS: 267.0 [M+H]+.

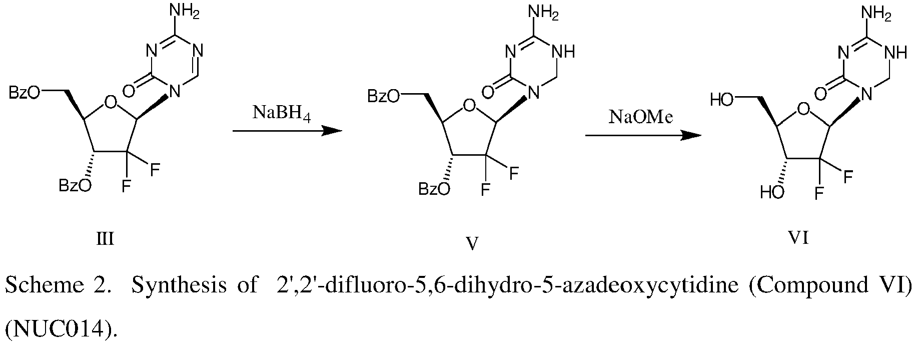

### EXAMPLE 3

### Preparation of 2'-Deoxy-2',2'-difluorozebularine (NUC019)

Preparation of 2'-deoxy-2',2'-difluorozebularine (Compound VIII) (NUC019) is shown in Scheme 3.
(1) The starting material 1-bromo-2-deoxy-2,2-difluoro-D-ribofuranosyl-3,5-dibenzoate (Compound I) was prepared as described in the Example 1.
(2) 2-Hydroxypyrimidine hydrochloride (0.57 g, 4.3 mmol) was suspended in hexamethyldisilazane (10 ml). The reaction mixture was heated at reflux for 1.5 h. The clear solution was decanted under argon, evaporated, coevaporated twice with dry xylene and vacuum dried.
(3) For glycosylation step, 1-bromo-2-deoxy-2,2-difluoro-D-ribofuranosyl-3,5-dibenzoate (Compound I) (1.20 g, 2.7 mmol), prepared as in Step (1), was dissolved in 20 ml of dichloroethane and transferred to the flask containing the silylated 2-hydroxypyrimidine. The reaction mixture was refluxed under argon for 16 hr, then dichloroethane was replaced with 10 mL of anisole, and the temperature was raised to 150 °C. The reaction mixture was stirred at 150 °C for 18 hr, cooed to room temperature, and the product, 1-β-(2'-deoxy-2',2'-difluoro-D-ribofuranosyl)-pyrimidine-2-one (Compound VII), was isolated as a mixture of β- and α-isomers (1:6) by flash chromatography on silica gel in methanol/chloroform (3:97). Yield: 0.09 g or 7%.
(4) 1-β-(2'-Deoxy-2',2'-difluoro-D-ribofuranosyl)-pyrimidine-2-one (Compound VII), prepared as in Steps (1) to (3), (85 mg, 6:1 mixture of α and β isomers) was dissolved in 2 ml of anhydrous MeOH and 1 M NaOMe in MeOH (0.1 ml) was added. After 1 h at 4 °C, the reaction mixture was evaporated and the deprotected isomers were isolated by RP HPLC on Gemini C18 5u (21.2x250 mm column) in 50 mM triethylammonium acetate (pH 7.5)-acetonitrile gradient. Immediately after separation fractions corresponding to β isomer were pooled and evaporated to yield 3.4 mg (7.4%) of 2'-deoxy-2',2'-difluorozebularine (Compound VIII).
   ¹H NMR in DMSO-d6 for compound VIII β-isomer: 8.66 ppm (dd, J₁=4 Hz, J2=2.4 Hz, 1H), 8.57 (dd, J₁=6.8 Hz, J2=2.4 Hz, 1H), 6.62 (dd, J₁=6.8 Hz, J2=4 Hz, 1H), 6.07 (t, J=7.2 Hz, 1H), 4.42-4.29 (m, 1H), 4.05-3.97 (m, 2H), 3.88-3.82 (m, 1H). ESI MS: 249 [M+H]⁺.

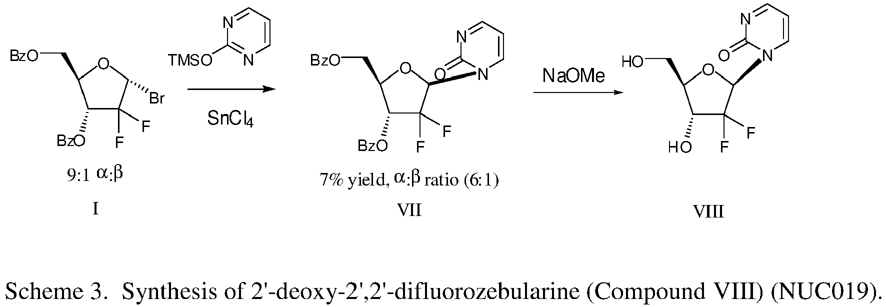

### EXAMPLE 4

### In vitro Cytotoxicity of Pyrimidine Analogs

In this example, the in vitro cytotoxicity, as measured by GI₅₀ (50% of growth inhibition), of the following pyrimidine analogs was measured using assays known in the art: gemcitabine, 5-azadeoxycytidine, 5-azacytidine, 2',2'-difluoro-5-azadeoxycytidine (NUC013), 5,6-dihydro-5-azadeoxycytidine, and 2'-deoxy-2',2'-difluorozebularine (NUC019).

The in vitro cytotoxicity was measured in the following cell lines: NCI-H460 (ATCC #HTB-177) (non-small cell lung), HCT-116 (ATCC #CCL-247) (colorectal), HL-60 (ATCC #CCL-240) (leukemia), and MDA-MB-231 (ATCC #HTB-26) (breast). The calculated GI₅₀ values are shown in Table 4.

**Table 4. Growth Inhibition (GI₅₀) Values (µM) for Selected Cell Lines.**

| Compound | NCI-H460 | MDA-MB-231 | HCT-116 | HL-60 |
|---|---|---|---|---|
| gemcitabine | 0.02 | 0.72 | 0.01 | 0.03 |
| 5-azadeoxycytidine | 13.04 | >31.25 | >31.25 | 0.27 |
| 5 -azacytidine | 0.43 | 1.64 | 0.62 | 0.44 |
| 2',2'-difluoro-5-azadeoxycytidine (NUC013) | 4.39 | >31.25 | 4.54 | 1.51 |
| 5,6-dihydro-5-azacytidine | 4.84 | >31.25 | 21.40 | 0.03 |
| 2'-deoxy-2',2'-difluorozebularine (NUC019) | 135.65 | 165.90 | 310.26 | -- |

### EXAMPLE 5

### In Vitro DNA Methyltransferase Inhibition

The activity of DNA methyltransferase (DNMT) was measured in the cancer cell line HCT-116 (ATCC #CCL-247) (colorectal) and the percent inhibition of DNMT resulting from treatment of the cells with 5-azacytidine (5-aza-C) or 2',2'-difluoro-5-azadeoxycytidine (Compound IV) (NUC013) was calculated. The assay was performed using the EpiQuick DNA Methyltransferase Activity/Inhibition Assay Kit (Epigentek, Brooklyn, NY). The HT-116 cells were incubated with 1 µM and 10 µM concentrations of each drug for a period of 24 hours. The percent inhibition of DNMT is shown in Table 5.

**Table 5. Percent Inhibition of DNMT**

| Experiment | 5-aza-C 1 µM | Compound IV (NUC013) 1µM | 5-aza-C 10 µM | Compound IV (NUC013) 10 µM |
|---|---|---|---|---|
| 1 | N/A | N/A | 30.41 | 28.20 |
| 2 | 36.76 | 61.73 | 59.90 | N/A |
| 3 | 9.94 | 35.77 | 41.26 | 46.51 |
| 4 | 10.96 | 62.63 | 74.22 | 70.75 |

The results in Table 8 demonstrate that 2',2'-difluoro-5-azadeoxycytidine (NUC013) is an inhibitor of DNA methyltransferase.

While the preferred embodiment of the invention has been illustrated and described, it will be appreciated that various changes can be made therein without departing from the scope of the invention.

## Claims

1. 2',2'-Difluoro-5-azadeoxycytidine, its anomers, and pharmaceutically acceptable salts thereof for use in the treatment of a solid tumor carcinoma.

2. 2',2'-Difluoro-5-azadeoxycytidine, its anomers, and pharmaceutically acceptable salts thereof for use according to claim 1, wherein the solid tumor carcinoma is selected from the group consisting of breast, non-small cell lung, colon, renal, ovarian, and colorectal carcinomas.

3. 2',2'-Difluoro-5-azadeoxycytidine, its anomers, and pharmaceutically acceptable salts thereof for use in the treatment of a hematologic malignancy.

4. 2',2'-Difluoro-5-azadeoxycytidine, its anomers, and pharmaceutically acceptable salts thereof for use according to claim 3, wherein the hematologic malignancy is a leukemia.

5. 2',2'-Difluoro-5-azadeoxycytidine, its anomers, and pharmaceutically acceptable salts thereof for use according to claim 4, wherein the leukemia is resistant to decitabine (5-azadeoxycytidine).

6. 2',2'-Difluoro-5,6-dihydro-5-azadeoxycytidine, its anomers, and pharmaceutically acceptable salts thereof.

7. A pharmaceutical composition comprising the compound of claim 6 and a pharmaceutically acceptable carrier.

8. The compound of claim 6 for use in the treatment of solid tumor carcinoma or a hematologic malignancy.

9. The compound of claim 6 for use according to claim 8, wherein
- the solid tumor carcinoma is selected from the group consisting of breast, non-small cell lung, and colon carcinomas; and
- the hematologic malignancy is leukemia.

10. 2'-Deoxy-2',2'-difluorozebularine, its anomers, and pharmaceutically acceptable salts thereof.

11. A pharmaceutical composition comprising the compound of claim 8 and a pharmaceutically acceptable carrier.

12. The compound of claim 10 for use in the treatment of solid tumor carcinoma or a hematologic malignancy.

13. The compound of claim 10 for use according to claim 12, wherein
- the solid tumor carcinoma is selected from the group consisting of breast, non-small cell lung, colon and colorectal carcinomas; and
- the hematologic malignancy is leukemia.

## Patentansprüche

1. 2',2'-Difluoro-5-azadesoxycytidin, dessen Anomere und pharmazeutisch akzeptable Salze davon, zur Verwendung bei der Behandlung eines soliden Tumorkarzinoms.

2. 2',2'-Difluoro-5-azadesoxycytidin, dessen Anomere und pharmazeutisch akzeptable Salze davon, zur Verwendung nach Anspruch 1, wobei das solide Tumorkarzinom aus der Gruppe ausgewählt ist, bestehend aus Brust-, nichtkleinzelligen Lungen-, Dickdarm-, Nieren-, Eierstock- und kolorektalen Karzinomen.

3. 2',2'-Difluoro-5-azadesoxycytidin, dessen Anomere und pharmazeutisch akzeptable Salze davon, zur Verwendung bei der Behandlung eines hämatologischen Malignoms.

4. 2',2'-Difluoro-5-azadesoxycytidin, dessen Anomere und pharmazeutisch akzeptable Salze davon, zur Verwendung nach Anspruch 3, wobei das hämatologische Malignom eine Leukämie ist.

5. 2',2'-Difluoro-5-azadesoxycytidin, dessen Anomere und pharmazeutisch akzeptable Salze davon, zur Verwendung nach Anspruch 4, wobei die Leukämie resistent gegen Decitabin-(5-azadesoxycytidin) ist.

6. 2',2'-Difluoro-5-azadesoxycytidin, dessen Anomere und pharmazeutisch akzeptable Salze davon.

7. Pharmazeutische Zusammensetzung, die die Verbindung von Anspruch 6 und einen pharmazeutisch akzeptablen Träger umfasst.

8. Verbindung nach Anspruch 6 zur Verwendung bei der Behandlung von soliden Tumorkarzinomen oder eines hämatologischen Malignoms.

9. Verbindung nach Anspruch 6 zur Verwendung nach Anspruch 8, wobei
- das solide Tumorkarzinom aus der Gruppe ausgewählt ist, bestehend aus Brust-, nichtkleinzelligen Lungen- und Dickdarmkarzinomen; und
- das hämatologische Malignom Leukämie ist.

10. 2'-Desoxy-2',2'-difluorozebularin, dessen Anomere und pharmazeutisch akzeptable Salze davon.

11. Pharmazeutische Zusammensetzung, umfassend die Verbindung nach Anspruch 8 und einen pharmazeutisch akzeptablen Träger.

12. Verbindung nach Anspruch 10 zur Verwendung bei der Behandlung von solidem Tumorkarzinom oder einem hämatologischen Malignom.

13. Verbindung nach Anspruch 10 zur Verwendung nach Anspruch 12, wobei
- das solide Tumorkarzinom aus der Gruppe ausgewählt ist, bestehend aus Brust-, nichtkleinzelligen Lungen- und Dickdarmkarzinomen; und
- das hämatologische Malignom Leukämie ist.

## Revendications

1. 2',2'-Difluoro-5-azadéoxycytidine, ses anomères, et ses sels pharmaceutiquement acceptables pour utilisation dans le traitement d'un carcinome à tumeur solide.

2. 2',2'-Difluoro-5-azadéoxycytidine, ses anomères et ses sels pharmaceutiquement acceptables pour utilisation selon la revendication 1, dans laquelle le carcinome à tumeur solide est choisi dans le groupe consistant en des carcinomes du sein, du poumon non à petites cellules, du côlon, du rein, des ovaires et du colorectum.

3. 2',2'-Difluoro-5-azadéoxycytidine, ses anomères, et ses sels pharmaceutiquement acceptables pour utilisation dans le traitement d'une hémopathie maligne.

4. 2',2'-Difluoro-5-azadéoxycytidine, ses anomères, et ses sels pharmaceutiquement acceptables pour utilisation selon la revendication 3, dans laquelle l'hémopathie maligne est une leucémie.

5. 2',2'-Difluoro-5-azadéoxycytidine, ses anomères, et ses sels pharmaceutiquement acceptables pour utilisation selon la revendication 4, dans laquelle la leucémie est résistante à la décitabine (5-azadéoxycytidine).

6. 2',2'-Difluoro-5,6-dihydro-5-azadéoxycytidine, ses anomères, et ses sels pharmaceutiquement acceptables.

7. Composition pharmaceutique comprenant le composé selon la revendication 6 et un support pharmaceutiquement acceptable.

8. Composé selon la revendication 6 pour son utilisation dans le traitement d'un carcinome à tumeur solide ou d'une hémopathie maligne.

9. Composé selon la revendication 6 pour utilisation selon la revendication 8, dans lequel
- le carcinome à tumeur solide est choisi dans le groupe consistant en des carcinomes du sein, du poumon non à petites cellules et du côlon ; et
- l'hémopathie maligne est une leucémie.

10. 2'-Déoxy-2',2'-difluorozébularine, ses anomères, et ses sels pharmaceutiquement acceptables.

11. Composition pharmaceutique comprenant le composé selon la revendication 8 et un support pharmaceutiquement acceptable.

12. Composé selon la revendication 10 pour utilisation dans le traitement d'un carcinome à tumeur solide ou d'une hémopathie maligne.

13. Composé selon la revendication 10 pour utilisation selon la revendication 12, dans lequel
- le carcinome à tumeur solide est choisi dans le groupe consistant en des carcinomes du sein, du poumon non à petites cellules, du côlon et du colorectum ; et
- l'hémopathie maligne est une leucémie.
